# EUROPEAN PATENT SPECIFICATION

(11) **EP 1 520 548 B1**
(45) Date of publication and mention of the grant of the patent: **22.11.2006**
(21) Application number: 04023372.8
(22) Date of filing: 30.09.2004
(51) Int. Cl.: A61B 19/00

(54) **Medical device supporting apparatus**
Stützgerät für chirurgische Instrumente
Support pour instruments chirurgicaux

(30) Priority: 03.10.2003 JP 2003345995; 13.02.2004 JP 2004036663
(43) Date of publication of application: 06.04.2005
(73) Proprietor: Olympus Corporation, Shibuya-ku, Tokyo (JP)
(72) Inventor: Otsuka, Satoshi, Mitaka-shi Tokyo (JP); Shinmura, Toru, Hachioji-shi Tokyo (JP); Yamashita, Tomoaki, Hachioji-shi Tokyo (JP)
(74) Representative: von Hellfeld, Axel

(56) References cited:
- EP-A- 0 293 760
- US-A- 5 271 384
- US-A- 5 597 146
- US-A1- 2002 177 857

## Description

### BACKGROUND OF THE INVENTION

### 1. Field of the Invention

The present invention relates to a medical device supporting apparatus used for positioning a medical device at an operative portion. For example, a medical device supporting apparatus of the invention is used for positioning an endoscope with respect to the operative portion in a surgical operation during a cranial nerve surgery.

### 2. Description of the Related Art

Generally, there is a medical device supporting apparatus for supporting a medical device disclosed in JP-A-2002-345831. The medical device supporting apparatus includes a plurality of arms connected via joints provided with brakes for locking and unlocking rotation thereof. The medical device supporting apparatus holds an endoscope by a holding device and locks the joints in a state of being faced toward the operative portion to be observed. Accordingly, since the visual field can be fixed without displacement, the operator can concentrate on the operation, thereby enabling an efficient operation.

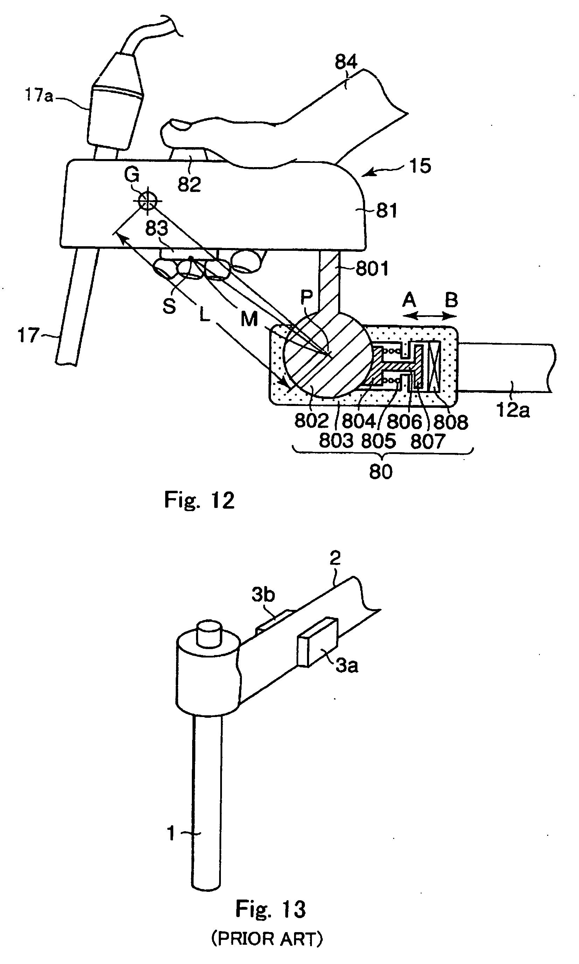

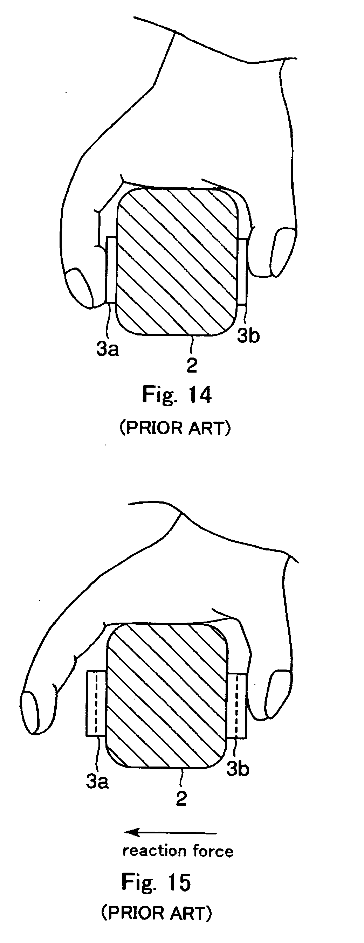

As shown in Fig. 13, such a medical device supporting apparatus includes a grip member 2 extending substantially orthogonal to an insertion axis of an endoscope 1 for locking and unlocking the joint brakes as a grip member to be provided in the vicinity of the holding device, to which the endoscope is attached, for moving the apparatus. Two operating switches 3a, 3b are provided substantially symmetrically with respect to the grip member 2. These two operation switches 3a, 3b are used in such a manner that the operator grips the grip member 2 as shown in Fig. 14, keeps pressing them simultaneously with his/her thumb and first finger to release the brakes, and unlocks the respective joints. Also, as shown in Fig. 15, the respective joints are locked in a state other than the state in which these two operating switches 3a, 3b are pressed simultaneously. Accordingly, the operator can concentrate on the operation without being absorbed in anxiety for erroneous unlocking of the brakes during surgical operation.

Also, the medical device supporting apparatus is required to have a substantial locking force in a state in which the brakes for the joints are locked so as not to be moved inadvertently in case where the operator happens to touch the arms, and simultaneously, to have a locking ability so that the operator can move the medical device in his/her hand lightly when they are unlocked and brought into a free state. In addition, it is also designed so that the brakes are maintained in a locked state in case of failure.

In the medical device supporting apparatus, in order to turn the operation switches 3a, 3b OFF to lock the respective joints after having moved the medical device, both of the two operation switches 3a, 3b are turned OFF. In this case, when the operator releases a force of his/her thumb and first finger which press the two operation switches 3a, 3b, which finger comes off the switch first is unknown. Therefore, there arises a time lag in the timing of being turned off between the operation switches 3a and 3b. A slight timing difference exists in the period from the timing when both of the operation switches 3a, 3b are turned OFF to the timing when the joint is locked, and hence the accuracy of joint lock is disadvantageously lowered.

In this arrangement, for example, when the operator's first finger comes off the operation switch 3a (3b) first, a reaction force generated when the first finger comes off is exerted in the operating direction, whereby the endoscope is moved and misalignment of the visual field may be resulted. In contrast, when his/her thumb comes off first from the switch, the endoscope is obliged to move in the opposite direction (See Fig. 15), and misalignment of the visual field in the opposite direction may be resulted. In this manner, if the visual field is displaced in the direction that the operator cannot predict, he/she has to align the position of the visual field again, and hence longer time is required for the surgical operation.

In addition, according to the medical device supporting apparatus described above, when it is applied in the field of a cranial nerve surgery for example, it is often moved in accordance with the progression of the surgical operation in an operating room in which various types of equipment are arranged. Therefore, there is a risk that somebody steps on the power cable when moving the apparatus including such equipment, which may result in disconnection of the power cable or failure of the brake control system. When disconnection of the power cable or failure of the brake system happens as described above, there arises a necessity to move the arm, which holds the medical device such as an endoscope, in a state in which the endoscope is inserted into the body cavity, and then pulled out from the body cavity. In this case, since the respective joints are in the locked state, it is necessary to move the arm against a locking force of the brake for the joint, which is disadvantageously hard to handle.

Another articulated device for supporting a medical instrument is described in European patent application EP 0 293 760 A2. With this device, which forms the preamble of claim 1, the position of the medical instrument is fixed under lightly-loaded restraint so that the user may adjust the instrument position with the application of a light force to the instrument. This has a disadvantage that the instrument may be inadvertently knocked out of position relatively easily by the user. Operation of this device is also sub-optimal.

### BRIEF SUMMARY OF THE INVENTION

The present invention provides a medical device supporting apparatus having the features recited in claim 1. Preferred features of the invention are recited in the dependent claims. The invention also provides a method of positioning a medical device (in three-dimensional space) with such an apparatus as defined in claim 17.

A medical device supporting apparatus according to the invention includes a supporting mechanism which can move three-dimensionally and lock a holding device for holding the medical device for observing or giving medical treatment to the operative portion, a control unit for controlling movement or locking of the holding device, and at least two final control elements for giving instructions for the operation to the control unit, and is characterized in that the amount of operating force of the respective final control element are differentiated.

In this arrangement, at least two final control elements being different in the amount of operating force, is turned OFF sequentially from the one having a larger operating force when the amount of operating force is reduced for releasing the operation. Therefore, the user (the operator) can recognize completion of operation of two final control elements reliably. Therefore, locking and unlocking operation of the medial treatment device with high degree of reliability and accuracy can be realized simply and easily.

Preferably, the holding member is restored from the moving state to the locked state by the control unit in a state in which one of the aforementioned two operating members is released.

When controlling the state of the holding members as described above, the operation of the operating member having the largest operating force is released first when the user weakens the force for releasing the operation of the operating member. In other words, the operating member which is released first is fixed, and hence when the operation of this specific operating member is released, the holding device is restored to the locked state. Therefore, the restoring operation from the moving state to the locked state is stabilized and is performed quickly.

A medical device supporting apparatus of the invention includes a supporting mechanism for supporting the holding device for holding the medical device for observing and giving medical treatment to the operative portion three-dimensionally so as to be movable and lockable, the supporting mechanism having a plurality of arms rotatably connected via joints which can be locked and unlocked of rotation, a control unit for controlling movement and locking of the holding device by locking and unlocking the rotation of the joints of the supporting mechanism, an input mechanism for giving instructions for the operation of the control unit, and a locking force adjusting mechanism for adjusting a locking force for locking the rotation of the joints.

In this arrangement, the medical device locked in position and supported by the supporting mechanism can adjust the movement of the joints by adjusting the force for locking the joints without performing the locking and unlocking operation of the joints of the supporting mechanism. Therefore, even in the case in which the locking state of the joints can hardly be released by the control unit, the supporting mechanism can be moved by weakening the locking force, so that the movement of the medical device including retraction is enabled.

### BRIEF DESCRIPTION OF THE SEVERAL VIEW OF THE DRAWINGS

These and other features, aspects, and advantages of the apparatus and methods of the present invention will become better understood with regard to the following description, appended claims, and accompanying drawings where:
Fig. 1 is a perspective view showing a structure of a medical device supporting apparatus according to a first embodiment of the present invention;
Fig. 2 is a partial cross-sectional view illustrating layout of first and second switch levers with respect to a holding device shown in Fig. 1;
Figs. 3A,3B are explanatory drawings illustrating a structure of joints in Fig. 1;
Fig. 4 is a cross-sectional view of a portion of the medical device supporting apparatus according to a second embodiment of the present invention;
Fig. 5 is a cross-sectional view illustrating a locking force adjusting mechanism disposed at the joints of the medical device supporting apparatus according to the second embodiment shown in Fig. 4;
Fig. 6 is a circuit diagram of a motor control system in Fig. 5;
Fig. 7 is a perspective view showing a structure of the medical device supporting apparatus according to a third embodiment of the invention;
Fig. 8 is a block diagram showing an air pressure control unit in Fig. 7;
Fig. 9 is a detailed drawing showing a stopper in Fig. 7;
Fig. 10 is a partial cross-sectional view showing a holding device in which an input portion in Fig. 7 is disposed;
Fig. 11 is a perspective view showing the structure of the medical device supporting apparatus according to a fourth embodiment of the invention;
Fig. 12 is a partial cross-sectional view of a portion in Fig. 11;
Fig. 13 is a drawing illustrating problems in the related art;
Fig. 14 is a drawing showing a positional relationship with respect to a switch in a holding state in Fig. 13; and
Fig. 15 is a drawing showing change-over operation of the switch in Fig. 13.

### DESCRIPTION OF THE PREFERRED EMBODIMENTS

Preferred embodiments of the invention will be described below with reference to the accompanying drawings.

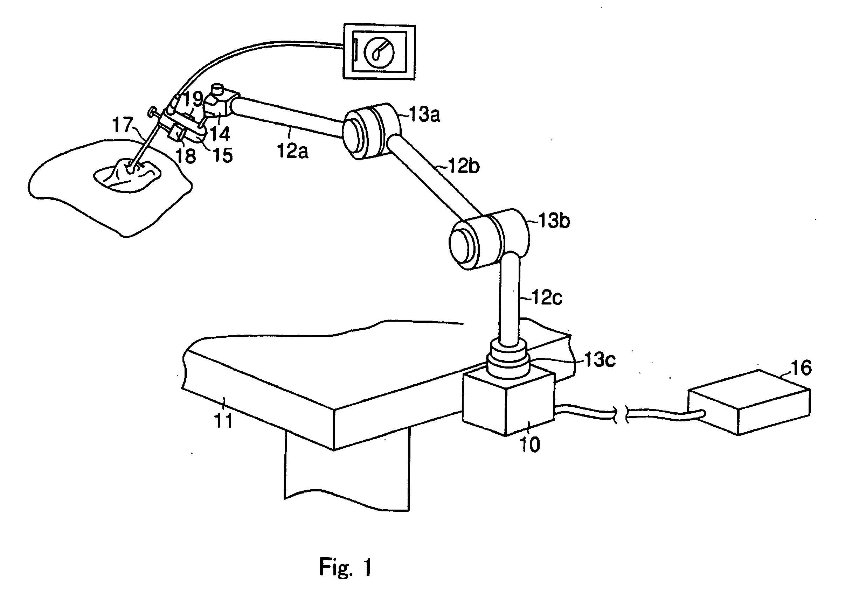

Fig. 1 shows a medical device supporting apparatus according to a first embodiment of the present invention. A supporting base 10 is detachably attached to a mounting body 11 such as a floor or a bed. Arms 12a, 12b, 12c are joined to the supporting base 10 via joints 13a, 13b, 13c in sequence. A holding device 15 for mounting a medical device is attached to the distal arm 12a via a ball joint 14. The ball joint 14 can include an electromagnet (not shown), which has an electromagnetic brake built therein. The electromagnet (not shown) is electrically connected to a control box 16 which constitutes a control unit, and is driven under the control of the control box 16. In other words, the electromagnet (not shown) performs locking and unlocking of the position of the ball joint 14 according to presence or absence of electric distribution.

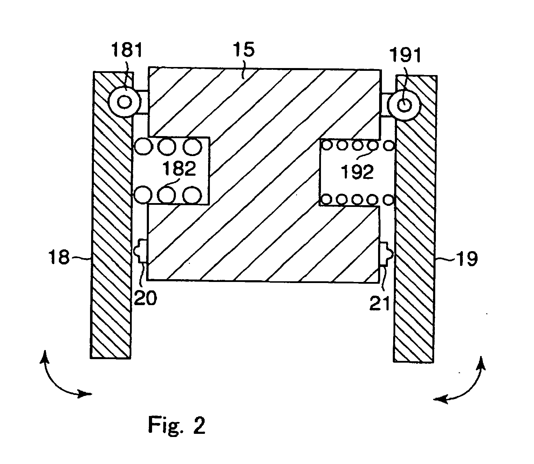

As a medical device for giving treatment or observing a patient, for example, an endoscope 17 is inserted into and supported by the holding device 15. The holding device 15 is provided with first and second switch levers 18, 19, which correspond to the final control elements, on both sides of the endoscope 17 so as to be operated in opposite directions. The first and second switch levers 18, 19 are rotatably supported via hinges 181, 191 at one end of each as shown in Fig. 2, and the proximal ends thereof are opposed to first and second micro switches 20, 21 disposed on the holding device 15. The first and second micro switches 20, 21 are electrically connected to the electromagnet (not shown) on the ball joint 14 and to electromagnets disposed on the joints 13a-13c, respectively (as discussed below).

The first and second switch levers 18, 19 are attached to the holding device 15 at midsections thereof via a first and second spring members 182, 192, and are restored to the initial positions (OFF positions of first and second micro switches 20, 21) by biasing forces of the first and second spring members 182, 192. The first and second spring members 182, 192 are set to different spring constants Ka, Kb, respectively, having a relation of Ka<Kb for example, and the amount of the operating force of the first and second switch levers 18, 19 are set based on the spring constants.

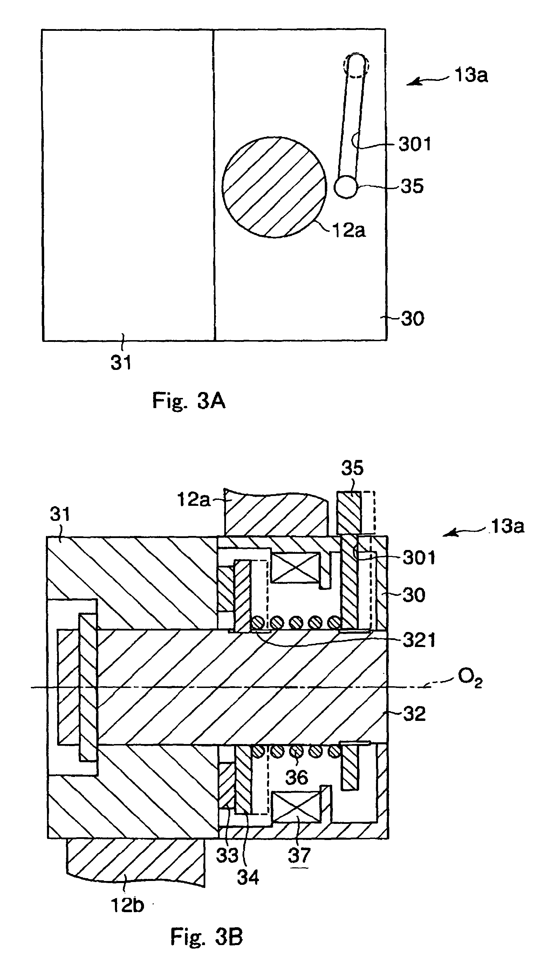

Referring now to Figs. 3A and 3B, the joints 13a, 13b, 13c will be described. However, since the joints are configured substantially in the same manner, the joint 13a will be described as a representative for convenience of explanation.

A joint member 31 is rotatably attached to a housing 30 about an axis O2 via a joint shaft 32. The distal end of the arm 12a is attached to the housing 30, and the distal end of the arm 12b is attached to the other joint member 31.

The joint member 31 is provided with a brake disk 33, and for example, an iron-made brake shoe 34 provided on the joint shaft 32 is opposed to the brake disk 33 so as to be capable of coming into and out of contact thereto. The brake shoe 34 is fitted into a key groove 321 provided on the joint shaft 32 so as to be capable of moving and is rotated about the axis O2 integrally with the joint shaft 32.

The housing 30 is provided with a cam 301, such as a slot, and a pin 35 which constitutes a rotational force adjusting mechanism is operably inserted into the cam 301. The operating tab 35 is rotatably supported at the distal end thereof about the joint shaft 32 and the rotary axis O2, and when it is urged to rotate about the rotary axis O2, it is moved along the cam 301 to the position indicated by broken lines in Figs. 3A and 3B.

A spring member 36 is engaged between the operating tab 35 and the brake shoe 34. The spring member 36 presses the brake shoe 34 against the brake disk 33 by its biasing force, and restricts the rotation of the joint member 31 by a frictional force generated at that time. Accordingly, when the operating tab 35 is moved along the cam 301, it varies the biasing force of the spring member 36 and varies a frictional force between the brake disk 33 and the brake shoe 34, so that the fixing force against the rotation of the joint member 31 is variably adjusted.

The housing 30 is provided with an electromagnet 37 so as to oppose the brake shoe 34. The electromagnet 37 pulls the brake shoe 34 by an electromagnetic force against the biasing force of the spring member 36. In other words, the electromagnet 37 is electrically connected to the control box 16, and turns the second micro switch 21 ON in conjunction with the operation of the second switch lever 19. When it is energized via the control box 16, the brake shoe 34 is pulled to the position indicated by the broken line in Fig. 3B and releases locking state of the joint member 31 with respect to the housing 30. Accordingly, the joint 13a is locked in a state in which the electromagnet 37 is not energized, and is unlocked in a state in which the electromagnet 37 is energized.

In this arrangement, during use, the operator operates the respective operating tabs 35 which are mounted to the respective joints 13a, 13b, 13c first to move a position shown by solid lines in Figs. 3A and 3B, where the biasing force of the spring member 36 becomes the maximum value. In this case, the locking force of the joints 13a, 13b, 13c are set to the maximum state.

In this case, the first and second micro switches 20, 21 are both in the OFF state, and hence the brake shoe 34 is pressed against the brake disk 33 by the spring member 36, whereby the joints 13a, 13b, 13c are locked against rotation by its frictional force. In other words, the arms 12a, 12b, and 12c, and the supporting base 10 do not move with respect to each other. The ball joint 14 is also locked against movement in the same manner, and the arm 12a and the holding device 15 are set to the locked state in which they do not move with respect each other.

Here, when it is necessary to move the arms 12a, 12b, 12c, the operator grips the holding device 15 and presses the first and second switch levers 18, 19. Then, the first and second micro switches 20, 21 are turned ON against the biasing forces of the spring members 182, 192, and its ON signal is supplied to the control box 16. The control box 16 supplies electric power to the respective electromagnets 37 of the joints 13a, 13b, 13c and the electromagnet (not shown) of the ball joint 14. Accordingly, the respective brake disks 33 of the joints 13a, 13b, 13c are moved to the position indicated by the broken line in Fig. 3B by the electromagnetic force of the electromagnet 37, and hence the frictional force between the brake disk 33 and the brake shoe 34 is removed, so that the locking against the rotation of the respective joints 13a, 13b, 13c is released.

At the same time, electric power is also supplied to the electromagnet (not shown) of the ball joint 14 so that locking of the hall joint 14 is released. Accordingly, the operator grips the holding device 15, moves the endoscope 17 (or other medical devices) to a desired position in the operative portion, and fixes the visual field which he or she wants to observe.

When locking the respective joints 13a, 13b, and 13c and the ball joint 14, the operator loosens the grip of the holding device 15. Then, the second switch lever 19 having the larger amount of operating force returns to the initial position by the second spring member 192 first, and then, the second micro switch 21 is turned OFF. Then, the control box 16 blocks the power supply to the electromagnets 37 of the joints 13a, 13b, 13c. Consequently, the brake shoe 34 is pressed against the brake disk 33 by the biasing force of the spring member 36, and the joints 13a, 13b, 13c are locked at the rotated positions, respectively.

At this time, the first switch lever 18 having the smaller amount of operating force does not return and is supported by a finger, the holding device 15 does not move and is held at the initial position. Then, when the operator further loosens the grip of the holding device 15, the first switch lever 18 having the smaller amount of the operating force is restored to its initial position by the first spring member 182, whereby the first micro switch 20 is turned OFF. Here, the control box 16 blocks the power supply to the electromagnet (not shown) of the ball joint 14. Consequently, the ball joint 14 is fixed and locked at its moved position, whereby the position setting of the endoscope 17 is completed.

In the case where the locking state of the respective joints cannot be released due to power outage or disconnection of the power cable with the endoscope 17 inserted in the operative portion during surgical operation, the operator rotates the operating tabs 35 of the respective joints 13a, 13b, 13c. Then the operating tab 35 is moved along the cam 301 to the position indicated by the broken lines in Figs. 3A and 3B. Accordingly, the biasing force of the spring member 36 is lowered, the contact pressure between the brake shoe 34 and the brake disk 33 is lowered, the frictional force therebetween is reduced, and the locking forces of the joints 13a, 13b, 13c are set so as to be capable of manual operation. In this case, the amount of locking force (frictional force) between the brake disk 33 and the brake shoe 34 is set to an extent in which the arms 12a, 12b, 12c do not move spontaneously, which is the amount of locking force in which the operator can move the joints 13a, 13b, 13c. In this state, the operator adjusts the movement of the arms 12a, 12b, 12c by a force larger than the amount of locking force, whereby the endoscope 17 at the holding device 15 can be withdrawn from the operative portion.

In this manner, the medical device supporting apparatus is configured in such a manner that the first and second switch levers 18, 19 having different amount of operating force are disposed correspondingly, and locking and unlocking of the arms 12a, 12b, 12c and the holding device 15 are performed in conjunction with the operation of the first and second switch levers 18, 19.

Accordingly, the operator can recognize completion of operation reliably by the switching operation of the first and second switch levers 18, 19 being turned off in sequence according to the amount of operation thereof, and thus locking and unlocking operation of the endoscope 17 with high reliability and accuracy can be realized simply and easily.

More specifically, the second micro switch 21 having a larger amount of operating force is turned OFF first via the second switch lever 19, and the first switch lever 18 of the first micro switch 20 having smaller amount of operating force does not return, and hence it can be supported by a finger. Therefore, the holding device 15 does not move with respect to the arms 12a, 12b, 12c, and a desired visual field of the endoscope (or adjustment of a medical device) can be easily fixed.

The operating tabs 35 for adjusting the locking force for the joints 13a, 13b, 13c respectively are adapted to be capable of being operable externally. In this arrangement, even when a failure such as disconnection of the cable or power outage happens during surgical operation, since the arms 12a, 12b, 12c can be moved easily by loosening the locking forces of the joints 13a, 13b, 13c by the operating tabs 35 during the surgical operation, movement of the endoscope 17 including retraction can easily be performed, whereby its handling property is improved.

A second embodiment of the present invention will now be described.

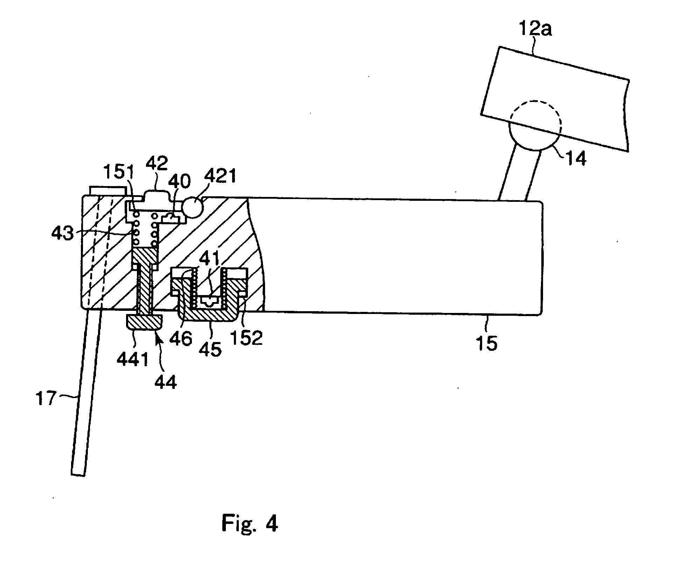

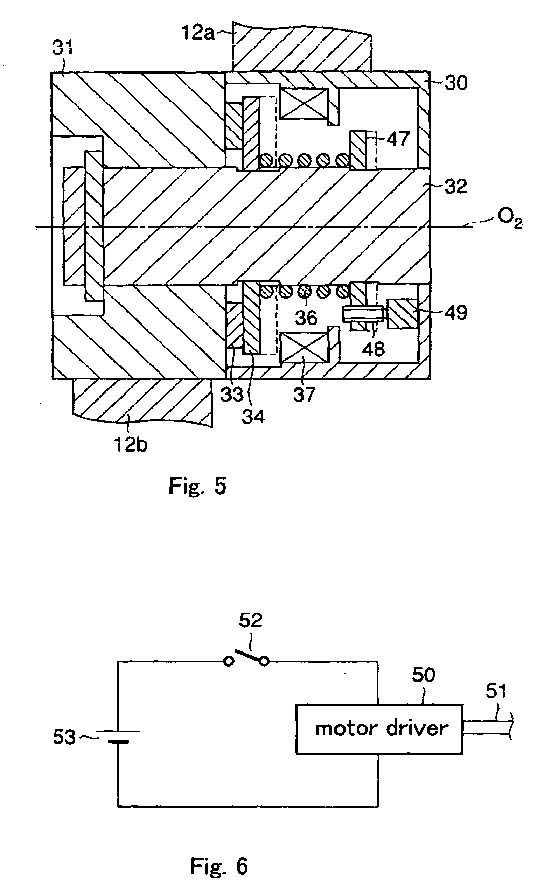

Fig. 4 and Fig. 5 show a medical device supporting apparatus according to the second embodiment of the invention, and include an emergency operating device in addition to the aforementioned first embodiment, which is expected to have the same effect as the first embodiment. Therefore, in Fig. 4 and Fig. 5, identical parts to Figs. 1 to 3 are represented by the identical numerals and detailed description will not be made.

In other words, for example, the endoscope 17 is detachably inserted into the holding device 15. At the position of the holding device 15 in the vicinity of the endoscope mounting position, there are provided recessed first and second switch storage portions 151, 152 so as to oppose each other, and first and second micro switches 40, 41 are stored and disposed in the first and second switch storage portions 151, 152 so as to be operated in the opposite direction. The first and second micro switches 40, 41 are electrically connected to the control box 16.

A switch lever 42 is rotatably provided in the first switch storage portion 151 via a hinge 421 so as to oppose the first micro switch 40, and a clockwise (the direction to turn ON the first micro switch 40) biasing force is exerted to the switch lever 42 via a first spring member 43. The proximal end of the first spring member 43 is adjustably engaged with the distal end of the operating force adjusting member 44. The operating force adjusting member 44 is supported at the midsection by the holding device 15 so as to be capable of screw-adjustment, and is operably provided with a final control element 441 at the proximal end so as to project from the holding device. Accordingly, the operating force adjusting member 44 is moved against the first spring member 43 by its rotating operation, and a biasing force of first spring member 43 is variably set to variably set the operating force of the switch lever 42. That is, the operating force adjusting member 44 is threadingly engaged with the holding device 15 such that it can be rotated to compress (and therefore preload) the first spring member 43.

On the other hand, a recessed switch cover 45 is provided on the second switch storage portion 152 via a spring member 46 at the position opposing to the second micro switch 41 so as to be capable of pressing operation.

The joints 13a, 13b, 13c can be configured as shown in Fig. 5, and a pressing force adjusting member 47 is engaged to the other end of the spring member 36 which is engaged with the brake shoe 34 at one end. The pressing force adjusting member 47 is disposed at the joint shaft 32 so as to be capable of moving in the axial direction, and an adjusting screw 48 is adjustably screwed at a predetermined position. The adjusting screw 48 is connected to the revolving shaft of a brake force adjusting drive motor 49 at a proximal end thereof, and when it is rotated by the drive motor 49, the pressing force adjusting member 47 is moved axially via the adjusting screw 48 according to the direction of rotation. Accordingly, the pressing force adjusting member 47 variably set a biasing force of the spring member 36 according to the moved position thereof to variably set the press-contact force between the brake shoe 34 and the brake disk 33.

The drive motor 49 is connected to a motor driver 50 which constitutes the emergency operating device as shown in Fig. 6 via a wiring cable 51. The motor driver 50 is provided in the control box 16 for example, and is connected to power supply 53 via an emergency moving switch 52. Accordingly, when the emergency moving switch 52 in the control box 16 is turned ON, the motor driver 50 receives a supply of ON signal, and outputs a drive signal to the drive motor 49 in response to this ON signal to drive and control the drive motor 49. Here, the pressing force adjusting member 47 is moved in the direction of O2 axis, the urging force of the spring member 36 is varied according to the moved position thereof, and the press-contact force between the brake shoe 34 and the brake disk 33 is adjusted. For example, the urging force of the spring member 36 of the pressing force adjusting member 47 is the smallest in a state in which the pressing force adjusting member 47 is moved to the position shown by a broken line in Fig. 5 and accommodates a predetermined failure.

In the structure describe above, the operator rotates the operating force adjusting member 44 before use, and varies the compression amount of the spring member 43 to adjust the amount of operating force of the switch lever 42 according to the taste of the operator, grips the holding device 15 so that his/her thumb is placed on the switch lever 42 and the first finger is placed to the switch cover 45 for pressing operation. Here, the first and second micro switches 40, 41 are turned ON against the urging force of the spring members 43, 46, and the respective ON signal is supplied to the control box 16. Then, the control box 16 supplies electric power to the electromagnets 37 of the joints 13a, 13b, 13c and the electromagnet (not shown) of the ball joint 14 of the holding device 15 which holds the endoscope 17, so that the respective joints 13a, 13b, 13c and the ball joint 14 are set to the rotatable state. In this state, the endoscope 17 is moved to a desired position in the operative portion, the visual field to be observed is fixed, and then, a force to grip the switch lever 42 and the switch cover 45 is loosened in the same manner to turn the first and second micro switches 40, 41 OFF in sequence, and the respective joints 13a, 13b, 13c and the holding device 15 are movably placed and fixed.

In this case, the amount of operating force to turn the first micro switch 40 ON is set to a larger value than that of the second micro switch 41, a force of the thumb is loosened first to turn the first micro switch 40 to OFF. In this case, since the switch cover 45 is not restored and is maintained in a state of being supported by the first finger, the holding device 15 is not moved.

In contrast, when the amount of operating force to turn the second micro switch 41 ON is set to a value larger than that of the first micro switch 40, a force of the first finger is loosen first to turn the second micro switch 41 OFF. In this case, the switch lever 42 is not restored and is supported by the thumb. Simultaneously, since the lower side of the holding device 15 is in the state of being supported by fingers other than the thumb and the first finger, the holding device 15 is not moved.

In the case where the electromagnet cannot be energized due to power outage or disconnection of the power cable during use, the emergency moving switch 52 of the control box 16 is pressed. Then, the ON signal of the emergency moving switch 52 is supplied to the motor driver 50. The motor driver 50 here drives the drive motors 49 provided in the respective joints 13a, 13b, 13c, and moves the pressing force adjusting member 47 to the position indicated by the broken line in Fig. 5 via the adjusting screw 48. Accordingly, the biasing force of the spring member 36 is reduced in comparison with the non-failure state, and a contact pressure force to press the brake disk 33 against the brake shoe 34 is set to a smaller value.

Although the amount of locking force between the brake disk 33 and the brake shoe 34 is maintained in an extent in which the arms 12a, 12b, 12c are not moved spontaneously, but the operator can move the joints 13a, 13b, 13c. The operator moves the arms 12a, 12b, 12c by the amount of force larger than that for locking the joints 13a, 13b, 13c to withdraw the endoscope 17 supported by the holding device 15 from the operative poriton.

According to the second embodiment, since the amount of operating force of the first micro switch 40 is adapted to be selectively variable, the amount of operating force can be set selectively according to the taste of the operator, and hence the handling property can be improved. Also, in this arrangement, even in the case of failure such as disconnection of an electric system such as a power supply system or power outage, the arms 12a, 12b, 12c themselves are prevented from moving spontaneously only by pressing the emergency moving switch 52 and, in addition, the locking force of joints 13a, 13b, 13c can be adjusted to an extent in which the operator can move the arms 12a, 12b, 12c. Therefore, quick support in the event of failure is achieved.

Subsequently, a third embodiment of the present invention will be described.

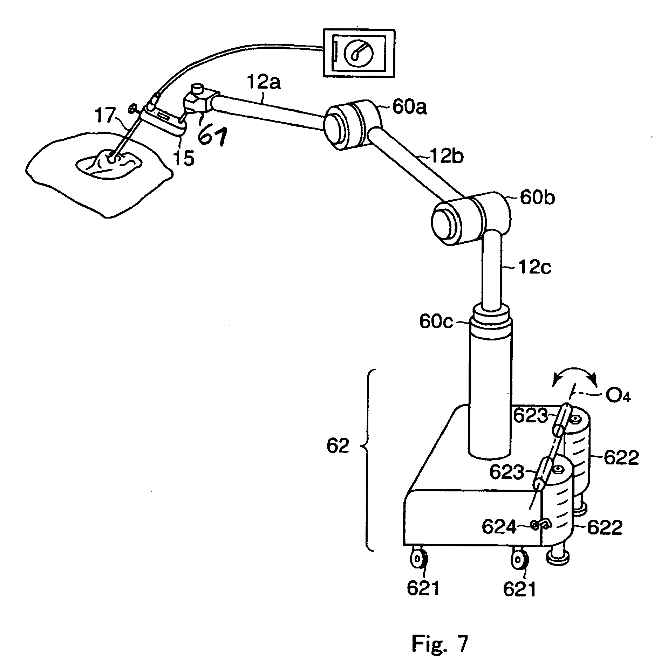

Fig. 7 shows a medical device supporting apparatus according to the third embodiment of the present invention, which is expected to have the same effect as the first and second embodiments. Therefore, in Fig. 7, identical parts to Fig. 1 are represented by the identical numerals and detailed description will not be made.

In other words, the aforementioned arms 12a, 12b, 12c are connected via a joint 60a, a joint 60b, and joint 60c, in which known fluid (pneumatic pressure) brakes are integrated, in sequence. The fluid brake is adapted to release the locking state of the brake when a pressure is applied. The holding device 15 is movable connected to the arm 12a via a ball joint 61 in which a fluid (pneumatic pressure) brake is integrated in the same manner. These fluid brakes are connected to a pneumatic pressure control device, described later, which is integrated in a carrier base 62 for supporting the arms 12a, 12b, 12c via piping (not shown).

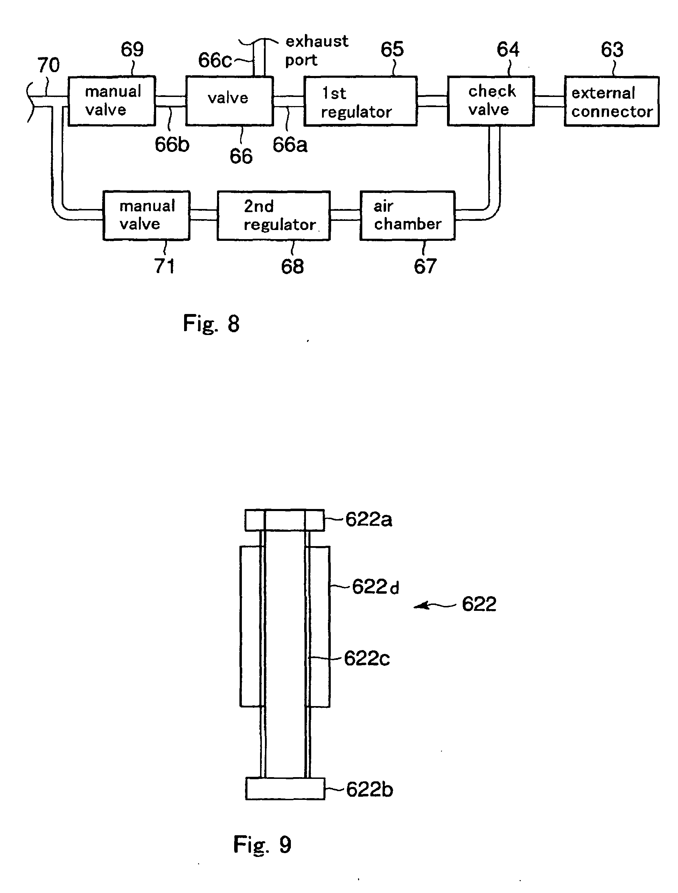

The pneumatic pressure control device is configured in such a manner that a fluid pressure source, not shown, such as a gas cylinder provided in a surgical operation room, is connected to an external connector 63; as shown in Fig. 8, and an input end of a check valve 64 is connected to the external connector 63. The check valve 64 is connected at an output end to an electromagnetic valve 66 via a first regulator 65 for adjusting the pressure, and at the other output end to a second regulator 68 for adjusting the pressure via an air chamber 67.

When no input signal is supplied to the electromagnetic valve 66 a first conduit line 66a is closed, and a second conduit line 66b and a third conduit line 66c, which is in communication with an exhaust port are communicated, while in a state in which an input signal is supplied thereto, the second conduit line 66b and the first conduit line 66a are brought into communication. Then, the second conduit line 66b of the electromagnetic valve 66 is connected to a conduit line 70 via a first manual valve 69, and is connected to the fluid brakes integrated in the joints 60a, 60b, 60c via the conduit line 70.

The second regulator 68 is connected to the second manual valve 71 to set fluid in the air chamber 67 at a pneumatic pressure providing the locking force to the airbrake to an extent that the arms 12a, 12b, 12c are not moved spontaneously, but may be moved manually by the operator, and output it to the conduit line 70 via the second manual valve 71.

The carrier base 62 is provided with carrier wheels 621 at four corners thereof, and for example, two stoppers 622, 622 are rotatably provided about a rotary axis O4 via hinges 623, 623 at a predetermined distance corresponding to the carrier wheels 621. The stoppers 622, 622 are disposed so as to be selectively rotatable via detent device 624 (in Fig. 7, only one of them is shown as a matter of convenience of the drawing).

The stoppers 622, 622 are provided with operating tabs 622a at the upper ends thereof as shown in Fig. 9, and with contact members 622b at the lower end thereof corresponding to the surface of installation such as a floor surface. Threaded portions 622c are provided at the midsection of the stopper 622, and the threaded portions 622c are attached to guiding members 622d so as to be adjustable by screwing. Accordingly, the stoppers 622, 622 are rotated by holding the operating tabs 622a by hand, so that the threaded portions 622c are moved in the vertical direction by being guided by the guiding members 622d.

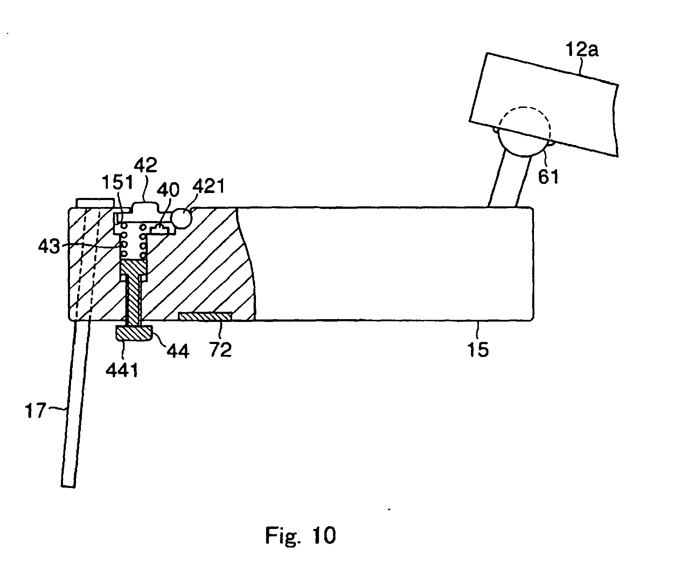

As shown in Fig. 10, the aforementioned holding device 15 is provided with a known photo interrupter 72 instead of the second micro switch 41 in the second embodiment. The photo interrupter 72 has two contact points on the back side thereof, and is adapted in such a manner that the two contact points are electrically short circuited when being touched to detect contact with the operator's hand.

The photo interrupter 72 and the first micro switch 40 of the holding device 15 are connected in series to a control circuit of the electromagnetic valve 66. The control circuit drives the electromagnetic valve 66 in a state in which the photo interrupter 72 and the first micro switch 40 are in the OFF state to supply air to the fluid brakes of the respective joints 60a, 60b, 60c, and the ball joint 61.

In this arrangement, before performing the surgical operation, an assistant or a nurse releases the detent device 624 of the stopper 622, and allows rotation about the rotary axis 04 via the hinge 623. In this state, whether the contact surface of the contact portion 622b of the stopper 622 which comes into contact with the floor is dirty is checked. When the contact surface of the contact portion 622b is dirty, the contact surface is cleaned in order to secure the fixing force. Then, the carrier base 62 is moved to a desired position using the carrier wheels 621, where the operating tab 622a of the stopper 622 is rotated to cause the contact portion 622b to be abutted against the floor. The carrier base 62 is positioned on the floor so as not to move when two of the carrier wheels 621 on the side of the stopper come apart from the floor.

When the operator grips the holding device 15 in order to move the holding device 15 to a desired position, his/her first finger and second finger touch the photo interrupter 72, and the photo interrupter 72 detects touch of the fingers. In this state, the operator presses the switch lever 42 with his/her thumb, and turns the first micro switch 40 ON. Then, the electromagnetic valve 66 of the aforementioned pneumatic pressure control device is activated and air is supplied to the fluid brakes of the respective joints 60a, 60b, 60c and the fluid brake of the ball joint 61, so that the brakes of the respective joints 60a, 60b, 60c and the ball joint 61 are released.

Then, when the holding device 15 is moved to a desired position, the operator loosens a force exerted by his/her thumb to turn the first micro switch 40 OFF. Then, the electromagnetic valve 66 closes the first conduit line 66a, and air supplied to the fluid brakes is discharged into the atmospheric air through the exhaust port from the third conduit line 66c, so that the brakes of the respective joints 60a, 60b, 60c and the ball joint 61 are fixed. In this case, although it takes slight time from the timing when the first micro switch 40 is turned OFF to the timing when the respective joints 60a, 60b, 60c and the ball joint 61 are fixed, the holding device 15 does not move because a gravitational force and a supporting force of his/her hand are in balance.

When a problem occurs in the electric system due to power outage or disconnection of the power supply cable in a state in which the endoscope 17 is inserted into the operative portion during surgical operation, the first manual valve 69 is closed first, and then the second manual valve 71 is opened to supply air to the fluid brakes. Consequently, the locking forces of the brakes of the respective joints 60a, 60b, 60c are such that the arms 12a, 12b, 12c do not move spontaneously, but the operator can move the arms 12a, 12b, 12c by hand. In this state, when the operator moves the arms 12a, 12b, 12c, the endoscope 17 of the holding device 15 is withdrawn from the operative portion.

In this manner, according to the third embodiment, since there is no mechanisms for weakening the locking forces of the brakes provided in the respective joints 60a, 60b, 60c, the respective joints 60a, 60b, 60c can be reduced in size. Therefore, since the force of inertia caused by the mass of the joints can be reduced when moving the arms, operability is improved.

According to the third embodiment, since the switching structure using the first micro switch 40 and the photo interrupter 72 is employed, there is no switching error due to a reaction force of the first micro switch 40, and hence the switching operation is achieved with high degree of accuracy.

Furthermore, according to the third embodiment, since the stopper 622 is rotatably provided on the carrier base 62, and the carrier base 62 is positioned and fixed by the stopper 622, a constantly high locking force is obtained by rotating the stopper 622 in the reverse direction for cleaning the contact portion 622b thereof.

Subsequently, a fourth embodiment of the present invention will be described.

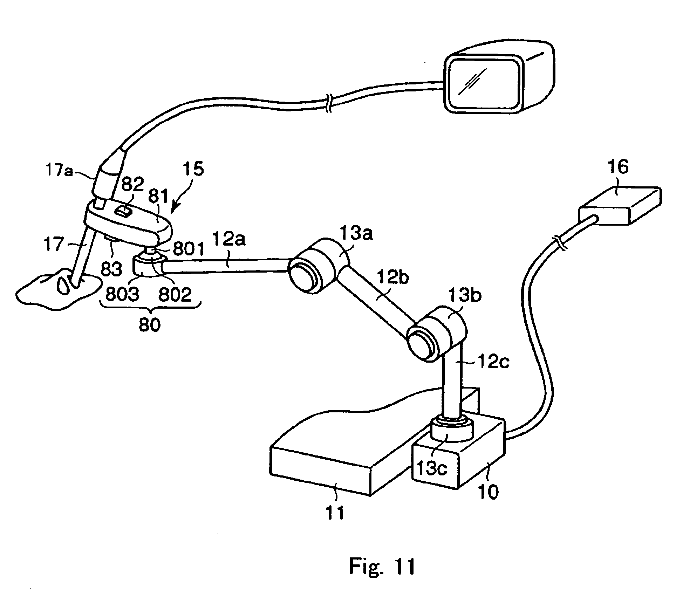

Fig. 11 and Fig. 12 show a medical device supporting apparatus according to the fourth embodiment of the present invention, in which further preferable effects are expected in comparison with the first to third embodiments. Therefore, in Fig. 11 and Fig. 12, identical parts to Fig. 1 are represented by the identical numerals and detailed description will not be made.

In the fourth embodiment, the distal portion of the arm 12a is attached to the holding unit 15, to which the endoscope 17 is detachably attached, on the lower side in the direction of a gravitational force (distal to an area of larger cross section 17a, e.g., a surface on a distal side of the endoscope) via a ball joint 80 so as to be capable of switching between the movable state and the locked state, and so as to be unbalanced with respect to the distal end of the arm 12a in the movable state.

The ball joint 80, as shown in Fig. 12, is attached at the proximal end of a rod 801 to the grip member 81 of the holding device 15 on the lower side in the direction of a gravitational force, and the distal end of the rod 801 is provided with a spherical member 802. The spherical member 802 is stored in the spherical seat housing 803 so as to be capable of moving and being locked. A pressing member 804 is stored in the spherical seat housing 803 so as to be capable of moving in the directions indicated by arrows A, B against the spherical member 802. The pressing member 804 is exerted with a biasing force in the direction indicated by the arrow A via a spring member 805, and a disk member 807 formed of magnetic material is attached to the proximal end thereof via a rod 806. The disk member 807 is disposed so as to oppose an electromagnet 808 at a predetermined distance.

The electromagnet 808 is connected to the control box 16 (see Fig. 11), and is driven and controlled via the control box 16. When the electromagnet 808 is driven via the control box 16, the electromagnet 808 moves the disk member 807 in the direction of the arrow B against the biasing force of the spring member 805, bringing the pressing member 804 away from the spherical member 802, setting the spherical member 802 in the spherical seat housing 803 so as to be capable of moving freely, and setting the distance between the arm 12a and the holding device 15 so as to be capable of moving. When the electromagnet 808 is stopped being driven, the electromagnet 808 allows the pressing member 804 to be biased and moved in the direction of the arrow A by the spring member 805. Accordingly, the pressing member 804 exerts a contact pressure against the spherical member 802 of the ball joint 80, and the ball joint 80 is positioned at the moved position, so that the arm 12a and the holding device 15 are fixed in position.

Assuming that the mass of the holding device 15 is J, the center of gravity thereof is G, and the length between the center of gravity G and the center position P of the ball joint 80 is L with the endoscope 17 inserted, the holding device 15 is assembled in an unbalanced state in which a moment load of LJ is generated with respect to the center position P depending on the mass J in a state in which the electromagnet 808 is driven and the spherical member is in a free state. The grip member 81 of the holding device 15 is provided with first and second switches 82, 83, which constitute a final control element of the input portion and are operated by different amounts of operating force, and are provided separately on the upper and lower ends in the direction of gravitational direction (corresponding to the direction of insertion of the endoscope 17) substantially parallel with each other. The first and second switches are electrically connected to the control box 16. The first switch 82 having a larger operating force is disposed on the upper side of the grip member 81 of the holding device 15, and, preferably, set to have the displacement of operation smaller than that of the second switch 83. The second witch 83 having the smaller operating force and, preferably, set to have a larger displacement in comparison with the first switch 82 is disposed on the lower side of the grip member 81 of the holding device 15.

Assuming that the distance between an operating portion S of the second switch 83 and the aforementioned center position P is M, the amount of operating force of the second switch 83 is set to a value smaller than a dropping moment (LJ/M) caused by aforementioned unbalance of the holding device 15. Accordingly, for example, during operation in the locked state, as an operator 84 gradually reduces the gripping force, the second switch 83 located on the lower side with respect to the gravitational force is in the state of being pressed by the finger of the operator 84 by the aforementioned dropping moment (□/M), and hence the first switch 82 is absolutely turned OFF prior to the second switch 83. Therefore, the operability of the first and second switches 82, 83 is enhanced, and hence adjustment of movement of the holding device 15 can be performed with a higher degree of accuracy.

Now, regarding the first and second switches 82, 83, the effect obtained by setting the amount of operation of the first switch 82 is set to a smaller value than the amount of operation of the second switch 83 will be described. For example, during operation in the locked state, when the operator 84 reduces the griping force gradually, as described above, the first switch 82 of the smaller amount of operation is immediately turned OFF, and thereafter, the second switch 83 is turned OFF, whereby the locking operation of the holding device 15 is completed. Therefore, by setting the amount of operation of the first switch 82 to a value as small as possible, the speed of turning-OFF operation of the first switch 82 is increased, and hence the speed-up of the locking operation is promoted.

In this arrangement, as regards the first and second switches 82, 83, when the operator 84 grips the grip member 81, the second switch 83 on the lower side is pressed first to output an ON-signal to the control box 16, and subsequently, the first switch 82 on the upper side is pressed to output an ON-signal to the control box 16 because of their amount of operating force. In this case, the control box 16 opens the spherical member 802 so as to be capable of moving with respect to the spherical seat housing 803 by driving the electromagnet 808 and attracting the pressing member 804 in the direction of the arrow B against the urging force of the spring member 805. Accordingly, the holding device 15 is capable of moving three-dimensionally with respect to the arm 12a, and moves the endoscope 17, which is inserted into the body cavity for example through an opening provided on the surface of the patient's body, in a desired position.

Subsequently, when positioning the endoscope 17, the operator 84 loosens his/her hand, which is gripping the grip member 81 of the holding device 15. At this time, the second switch 83 of the grip member 81 of the holding device 15 is released by the amount of operating force smaller than LJ/M, because the second switch 83 is in a state in which the moment load of LJ/M is provided to the operating portion S by the mass of the holding device 15 including the endoscope 17 as described above. In other words, in order to lock the endoscope 17 at a desired position, since the grip member 81 of the holding device 15 tends to drop because of the unbalanced state of the holding device 15, it is supported by the operating portion S of the second switch 83 on the lower side, and hence the first switch 82 on the upper side is turned OFF first.

At this time, the control box 16 stops driving the electromagnet 808. Then, the pressing member 804 of the ball joint 80 is urged in the direction of the arrow A by an urging force of the spring member 805 and is brought into press-contact with the spherical member 802, whereby the spherical member 802 is positioned with respect to the spherical seat housing 803, so that the holding device 15 is positioned and locked with respect to the arm 12a.

In this manner, in the fourth embodiment, the lower side in the direction of gravitational force of the holding device 15, which holds the endoscope 17, is assembled to the arm 12a, which is provided so as to be capable of moving and locking three-dimensionally, via the ball joint 80, which is capable of moving and locking, to dispose the holding device 15 in the unbalanced manner in a movable state, and the first and second switches 82, 83 operated by different amount of operating force are provided on both sides of the grip member 81 of the holding device 15 in the direction of the gravitational force of the holding device 15.

In this arrangement, since the holding device 15 is in the unbalanced state in a state in which the endoscope 17 is moved to a desired position by gripping the grip member 81 of the holding device 15 and moving the same with respect to the arm 12a, as the operator 84, griping the grip member 81 so as to prevent from dropping, loosens the gripping force gradually, the first switch 82 is consequently released first, then the second switch 83 is subsequently released. Therefore, the distal end of the endoscope 17 inserted into the holding device 15 is prevented from being displaced, and accurate positioning and locking at a desired position are achieved.

In this arrangement, the unbalanced structure in which the lower side in the direction of the gravitational force of the holding device 15 according to the fourth embodiment is assembled to the arm 12a so as to be capable of moving and locking via the ball joint 80, and in this movable state, the holding device 15 is supported in the unbalanced manner with respect to the arm 12a is also applicable to the first to third embodiments described above, and substantially the same effect can be expected.

Although the case in which the lower side surface in the direction of the gravitational force of the holding device 15 is attached to the arm 12a via the ball joint 80 so that the holding device 15 is disposed in an unbalanced manner in the movable state has been described in the fourth embodiment, it is not limited thereto, and various unbalanced structures may be employed.

Although the case in which the endoscope is used as the medical device has been described in the respective embodiments, it is not limited thereto, and may be used as a supporting structure for the medical device including various treatment devices, and substantially the same effect can be expected.

Although the case in which the invention is applied to the three-joint arm structure has been described in the respective embodiments, not limited to the number of arms, it may be applied to various types of arm structures, and substantially the same effects can be expected.

While there has been shown and described what is considered to be preferred embodiments of the invention, it will, of course, be understood that various modifications and changes in form or detail could readily be made without departing from the spirit of the invention. It is therefore intended that the invention be not limited to the exact form described and illustrated, but should be constructed to cover all modifications that may fall within the scope of the appended claims.

## Claims

1. A medical device supporting apparatus comprising:
a holding device (15) for holding a medical device (17);
a supporting mechanism which supports the holding device (15) for moving and locking the holding device (15) in a three-dimensional space;
a control unit (16) provided on the supporting mechanism and being capable of controlling movement and locking of the holding device (15); and
an input portion having at least two final control elements (18, 19; 40, 41; 82, 83) for operating the control unit (16),
**characterized in that** the at least two final control elements (18, 19; 40, 41; 82, 83) of the input portion are set to be operated by different amounts of operating force, such that the final control elements (18, 19; 40, 41; 82, 83) are adapted to sequentially operate the control unit (16) for movement and locking of the holding device (15) according to the amount of operating force.

2. A medical device supporting apparatus according to Claim 1, wherein the at least two final control elements (18, 19; 40, 41; 82, 83) of the input portion are disposed so that their operating directions with respect to the holding device (15) are set to be substantially opposite to each other.

3. A medical device supporting apparatus according to Claim 1, wherein one (18) of the at least two final control elements (18, 19) of the input portion is disposed on a first side of the holding device (15) and another (19) of the at least two final control elements (18, 19) is disposed on a second side of the holding member (15) such that operating directions of the at least two final control elements (18, 19) are set to be substantially opposite to each other.

4. A medical device supporting apparatus according to Claim 1, wherein the two final control elements (18, 19; 40, 41; 82, 83) of the input portion are disposed on a plane including a medical device mounting portion of the holding device (15) and an axis of the medical device (17) mounted to the medical device mounting portion extending in a direction of insertion.

5. A medical device supporting apparatus according to Claim 1, wherein the two final control elements (18, 19; 40, 41; 82, 83) of the input portion are disposed at both sides of the holding device (15) on a plane including a medical device mounting portion of the holding device (15) and an axis of the medical device (17) mounted to the medical device mounting portion extending in a direction of insertion.

6. A medical device supporting apparatus according to Claim 5, wherein one (18) of the at least two final control elements (18, 19) of the input portion, which is disposed on a side of the holding device (15) where the medical device (17) is inserted, is set to have a smaller amount of operating force than another (19) of the at least two final control elements (18, 19).

7. A medical device supporting apparatus according to Claim 1, further comprising an adjusting mechanism (43, 44) for adjusting the amount of operating force of at least one of the two final control elements (18, 19; 40, 41; 82, 83) of the input portion.

8. A medical device supporting apparatus according to Claim 1, wherein the at least two final control elements (18, 19; 40, 41; 82, 83) of the input portion are disposed symmetrically with respect to a plane including a medical device mounting portion and an axis of operating equipment attached to the medical device mounting portion extending in a direction of insertion.

9. A medical device supporting apparatus according to Claim 1, wherein at least one of the at least two final control elements (18, 19; 40, 41; 82, 83) of the input portion is a photo interrupter (72).

10. A medical device supporting apparatus according to Claim 1, wherein the supporting mechanism (80) supports the holding device (15) in an unbalanced state when the holding device (15) is in a movable state, and wherein the at least two final control elements (82, 83) are provided on both sides of the holding device (15) in the direction of a gravitational force of the holding device (15).

11. A medical device supporting apparatus according to Claim 10, wherein an operating force of one (83) of the at least two final control elements (82, 83) of the input portion located on a lower side of the holding device (15) in the direction of gravitational force is smaller than that of another (82) of the at least two final control elements (82, 83) located on an upper side of the holding device (15).

12. A medical device supporting apparatus according to Claim 10, wherein the amount of operation of one (82) of the at least two final control elements of the input portion located on an upper side of the holding device (15) in the direction of gravitational force is smaller than that of another (83) of the at least two final control elements (82, 83) located on a lower side of the holding device (15).

13. A medical device supporting apparatus according to Claim 10, wherein the amount of operating force of one (83) of the at least two final control elements (82, 83) of the input portion located on a lower side of the holding device (15) in the gravitational direction is smaller than a drop moment due to the unbalanced state of the holding device (15).

14. A medical device supporting apparatus according to Claim 10, wherein the supporting mechanism comprises a plurality of joints (13) and arms (12) for positioning the holding device (15) in three-dimensional space, and further comprising:
a brake mechanism (33, 34) provided on at least one of the joints (13a, 13b, 13c) for switching the at least one joint between a movable state and a locked state,
wherein the control unit (16) is adapted for controlling the brake mechanism (33, 34), and wherein the plurality of final control elements (82, 83) are adapted for giving instructions to the control unit (16) for making a state of the brake mechanism (33, 34) switch.

15. A medical device supporting apparatus according to Claim 14, wherein the control unit (16) makes the brake mechanism (33, 34) switch the joint (13) between the movable state and the locked state when operation is released by one of the plurality of final control elements (82, 83).

16. A medical device supporting apparatus according to Claim 1, wherein the supporting mechanism comprises a plurality of joints (13) and arms (12) for positioning the holding device (15) in three-dimensional space, and the medical device supporting apparatus further comprises:
a brake mechanism (33, 34) provided on at least one of the joints for switching the joint (13) between a movable state and a locked state; and
a mechanism (35) for manually switching the joint from the locked state to the movable state.

## Patentansprüche

1. Stützvorrichtung für medizintechnische Geräte, umfassend:
eine Haltevorrichtung (15) zum Halten eines medizintechnischen Geräts (17);
einen Stützmechanismus, welcher die Haltevorrichtung (15) stützt, zum Bewegen und Arretieren der Haltevorrichtung (15) in einem dreidimensionalen Raum;
eine Steuereinheit (16), die am Stützmechanismus vorgesehen ist und in der Lage ist, die Bewegung und die Arretierung der Haltevorrichtung (15) zu steuern;
einen Eingabeteil mit mindestens zwei Stellantrieben (18, 19; 40, 41; 82, 83) zum Betätigen der Steuereinheit (16),
**dadurch gekennzeichnet, dass** die mindestens zwei Stellantriebe (18, 19; 40, 41; 82, 83) des Eingabeteils dazu eingerichtet sind, durch unterschiedliche Stärken der Betätigungskraft betätigt zu werden, so dass die Stellantriebe (18, 19; 40, 41; 82, 83) daran angepasst sind, die Steuereinheit (16) zur Bewegung und Arretierung der Haltevorrichtung (15) gemäß der Stärke der Betätigungskraft sequentiell zu betätigen.

2. Stützvorrichtung für medizintechnische Geräte nach Anspruch 1, wobei die mindestens zwei Stellantriebe (18, 19; 40, 41; 82, 83) des Eingabeteils so angeordnet sind, das ihre Betätigungsrichtungen in Bezug auf die Haltevorrichtung (15) im wesentlichen entgegengesetzt zueinander eingerichtet sind.

3. Stützvorrichtung für medizintechnische Geräte nach Anspruch 1, wobei einer (18) der mindestens zwei Stellantriebe (18, 19) des Eingabeteils an einer ersten Seite der Haltevorrichtung (15) angeordnet ist und ein anderer (19) der mindestens zwei Stellantriebe (18, 19) an einer zweiten Seite der Haltevorrichtung (15) angeordnet ist, so dass Betätigungsrichtungen der mindestens zwei Stellantriebe (18, 19) im wesentlichen entgegengesetzt zueinander eingerichtet sind.

4. Stützvorrichtung für medizintechnische Geräte nach Anspruch 1, wobei die zwei Stellantriebe (18, 19; 40, 41; 82, 83) des Eingabeteils an einer Ebene angeordnet sind, welche einen Befestigungsteil für medizintechnische Geräte der Haltevorrichtung (15) und eine Achse des medizintechnischen Geräts (17), das am Befestigungsteil für medizintechnische Geräte befestigt ist, beinhaltet, die sich in einer Einführungsrichtung erstreckt.

5. Stützvorrichtung für medizintechnische Geräte nach Anspruch 1, wobei die zwei Stellantriebe (18, 19; 40, 41; 82, 83) des Eingabeteils an beiden Seiten der Haltevorrichtung (15) an einer Ebene angeordnet sind, welche einen Befestigungsteil für medizintechnische Geräte der Haltevorrichtung (15) und eine Achse des medizintechnischen Geräts (17), das am Befestigungsteil für medizintechnische Geräte befestigt ist, beinhaltet, die sich in einer Einführungsrichtung erstreckt.

6. Stützvorrichtung für medizintechnische Geräte nach Anspruch 5, wobei einer (18) der mindestens zwei Stellantriebe (18, 19) des Eingabeteils, welcher an einer Seite der Haltevorrichtung (15) angeordnet ist, wo das medizintechnische Gerät (17) eingeführt wird, dazu eingerichtet ist, eine geringere Stärke der Betätigungskraft als der andere (19) der mindestens zwei Stellantriebe (18, 19) aufzuweisen.

7. Stützvorrichtung für medizintechnische Geräte nach Anspruch 1, ferner umfassend einen Nachstellmechanismus (43, 44) zum Nachstellen der Stärke der Betätigungskraft mindestens eines der zwei Stellantriebe (18, 19; 40, 41; 82, 83) des Eingabeteils.

8. Stützvorrichtung für medizintechnische Geräte nach Anspruch 1, wobei die mindestens zwei Stellantriebe (18, 19; 40, 41; 82, 83) des Eingabeteils symmetrisch in Bezug auf eine Ebene angeordnet sind, welche einen Befestigungsteil für medizintechnische Geräte und eine Achse der Betriebseinrichtung, die am Befestigungsteil für medizintechnische Geräte befestigt ist, beinhaltet, die sich in einer Einführungsrichtung erstreckt.

9. Stützvorrichtung für medizintechnische Geräte nach Anspruch 1, wobei mindestens einer (18) der mindestens zwei Stellantriebe (18, 19; 40, 41; 82, 83) des Eingabeteils ein Lichtunterbrecher (72) ist.

10. Stützvorrichtung für medizintechnische Geräte nach Anspruch 1, wobei der Stützmechanismus (80) die Haltevorrichtung (15) in einem Ungleichgewichtszustand stützt, wenn die Haltevorrichtung (15) sich in einem bewegbaren Zustand befindet, und wobei die mindestens zwei Stellantriebe (82, 83) an beiden Seiten der Haltevorrichtung (15) in der Richtung einer Schwerkraft der Haltevorrichtung (15) vorgesehen sind.

11. Stützvorrichtung für medizintechnische Geräte nach Anspruch 10, wobei die Betätigungskraft eines (83) der mindestens zwei Stellantriebe (82, 83) des Eingabeteils, der sich an einer Unterseite der Haltevorrichtung (15) in Richtung der Schwerkraft befindet, kleiner ist als diejenige des anderen (82) der mindestens zwei Stellantriebe (82, 83), der an einer Oberseite der Haltevorrichtung (15) positioniert ist.

12. Stützvorrichtung für medizintechnische Geräte nach Anspruch 10, wobei die Betätigungskraft eines (82) der mindestens zwei Stellantriebe (82, 83) des Eingabeteils, der sich an einer Oberseite der Haltevorrichtung (15) in Richtung der Schwerkraft befindet, kleiner ist als diejenige des anderen (83) der mindestens zwei Stellantriebe (82, 83), der an einer Unterseite der Haltevorrichtung positioniert ist.

13. Stützvorrichtung für medizintechnische Geräte nach Anspruch 10, wobei die Betätigungskraft eines (83) der mindestens zwei Stellantriebe (82, 83) des Eingabeteils, der an einer Unterseite der Haltevorrichtung (15) in Richtung der Schwerkraft positioniert ist, kleiner ist als ein Fallmoment aufgrund des Ungleichgewichtszustands der Haltevorrichtung (15).

14. Stützvorrichtung für medizintechnische Geräte nach Anspruch 10, wobei der Stützmechanismus eine Vielzahl von Gelenken (13) und Auslegern (12) zum Positionieren der Haltevorrichtung (15) im dreidimensionalen Raum umfasst und ferner aufweist:
einen Bremsmechanismus (33, 34), der an zumindest einem der Gelenke (13a, 13b, 13c) zum Umschalten des mindestens einen Gelenks zwischen einem bewegbaren Zustand und einem arretierten Zustand vorgesehen ist,
wobei die Steuereinheit (16) angepasst ist an ein Steuern des Bremsmechanismus' (33, 34), und wobei die Vielzahl der Stellantriebe (82, 83) daran angepasst ist, Anweisungen an die Steuereinheit (16) auszugeben, um diese dazu zu bringen, einen Zustand des Bremsmechanismus' (33, 34) umzuschalten.

15. Stützvorrichtung für medizintechnische Geräte nach Anspruch 14, wobei die Steuereinheit (16) den Bremsmechanismus' (33, 34) zu bringt, das Gelenk (13) zwischen dem bewegbaren Zustand und dem arretierten Zustand umzuschalten, wenn die Betätigung durch einen der Vielzahl der Stellantriebe (82, 83) freigegeben wird.

16. Stützvorrichtung für medizintechnische Geräte nach Anspruch 1, wobei der Stützmechanismus eine Vielzahl von Gelenken (13) und Auslegern (12) zum Positionieren der Haltevorrichtung (15) im dreidimensionalen Raum umfasst, und die Stützvorrichtung für medizintechnische Geräte ferner aufweist::
einen Bremsmechanismus (33, 34), der an zumindest einem der Gelenke zum Umschalten des Gelenks (13) zwischen einem bewegbaren Zustand und einem arretierten Zustand vorgesehen ist; und
einen Mechanismus (35) zum händischen Umschalten des Gelenks aus dem arretierten Zustand in den bewegbaren Zustand.

## Revendications

1. Appareil de support de dispositif médical comprenant :
un dispositif de maintien (15) pour maintenir un dispositif médical (17) ;
un mécanisme de support qui supporte le dispositif de maintien (15) pour déplacer et verrouiller le dispositif de maintien (15) dans un espace tridimensionnel ;
un module de commande (16) disposé sur le mécanisme de support et étant capable de commander le mouvement et le verrouillage du dispositif de maintien (15) ; et
une partie d'entrée ayant au moins deux éléments de commande finals (18, 19 ; 40, 41 ; 82, 83) pour opérer le module de commande (16),
**caractérisé en ce que** les au moins deux éléments de commande finals (18, 19 ; 40, 41 ; 82, 83) de la partie d'entrée sont définis pour être opérés par différentes quantités de force d'opération, de telle sorte que les éléments de commande finals (18, 19 ; 40, 41 ; 82, 83) sont appropriés pour opérer séquentiellement le module de commande (16) pour le mouvement et le verrouillage du dispositif de maintien (15) selon la quantité de force d'opération.

2. Appareil de support de dispositif médical selon la revendication 1, dans lequel les au moins deux éléments de commande finals (18, 19 ; 40, 41 ; 82, 83) de la partie d'entrée sont disposés de telle sorte que leurs directions d'opération par rapport au dispositif de maintien (15) sont définies pour être substantiellement opposées l'une à l'autre.

3. Appareil de support de dispositif médical selon la revendication 1, dans lequel un (18) des au moins deux éléments de commande finals (18, 19) de la partie d'entrée est disposé sur un premier côté du dispositif de maintien (15) et un autre (19) des au moins deux éléments de commande finals (18, 19) est disposé sur un second côté du dispositif de maintien (15) de telle sorte que les directions d'opération des au moins deux éléments de commande finals (18, 19) sont définies pour être substantiellement opposées l'une à l'autre.

4. Appareil de support de dispositif médical selon la revendication 1, dans lequel les au moins deux éléments de commande finals (18, 19 ; 40, 41 ; 82, 83) de la partie d'entrée sont disposés sur un plan incluant une partie de montage de dispositif médical du dispositif de maintien (15) et un axe du dispositif médical (17) monté sur la partie de montage de dispositif médical s'étendant dans une direction d'insertion.

5. Appareil de support de dispositif médical selon la revendication 1, dans lequel les au moins deux éléments de commande finals (18, 19 ; 40, 41 ; 82, 83) de la partie d'entrée sont disposés des deux côtés du dispositif de maintien (15) sur un plan incluant une partie de montage de dispositif médical du dispositif de maintien (15) et un axe du dispositif médical (17) monté sur la partie de montage de dispositif médical s'étendant dans une direction d'insertion.

6. Appareil de support de dispositif médical selon la revendication 5, dans lequel un (18) des au moins deux éléments de commande finals (18, 19) de la partie d'entrée, qui est disposé sur un côté du dispositif de maintien (15) où le dispositif médical (17) est inséré, est défini pour avoir une plus petite quantité de force d'opération qu'un autre (19) des au moins deux éléments de commande finals (18, 19).

7. Appareil de support de dispositif médical selon la revendication 1, comprenant en outre un mécanisme d'ajustement (43, 44) pour ajuster la quantité de force d'opération d'au moins un des deux éléments de commande finals (18, 19 ; 40, 41 ; 82, 83) de la partie d'entrée.

8. Appareil de support de dispositif médical selon la revendication 1, dans lequel les au moins deux éléments de commande finals (18, 19 ; 40, 41 ; 82, 83) de la partie d'entrée sont disposés symétriquement par rapport à un plan incluant une partie de montage de dispositif médical et un axe d'équipement opérationnel monté sur la partie de montage de dispositif médical s'étendant dans une direction d'insertion.

9. Appareil de support de dispositif médical selon la revendication 1, dans lequel au moins un des au moins deux éléments de commande finals (18, 19 ; 40, 41 ; 82, 83) de la partie d'entrée est un photo-interrupteur (72).

10. Appareil de support de dispositif médical selon la revendication 1, dans lequel le mécanisme de support (80) supporte le dispositif de maintien (15) en un état non équilibré lorsque le dispositif de maintien (15) est en un état mobile, et dans lequel les au moins deux éléments de commande finals (82, 83) sont disposés des deux côtés du dispositif de maintien (15) dans la direction d'une force de gravitation du dispositif de maintien (15).

11. Appareil de support de dispositif médical selon la revendication 10, dans lequel une force d'opération d'un (83) des au moins deux éléments de commande finals (82, 83) de la partie d'entrée situé sur un côté inférieur du dispositif de maintien (15) dans la direction de la force de gravitation est plus petite que celle d'un autre (82) des au moins deux éléments de commande finals (82, 83) situé sur un côté supérieur du dispositif de maintien (15).

12. Appareil de support de dispositif médical selon la revendication 10, dans lequel la quantité d'opération d'un (82) des au moins deux éléments de commande finals (82, 83) de la partie d'entrée situé sur un côté supérieur du dispositif de maintien (15) dans la direction de la force de gravitation est plus petite que celle d'un autre (83) des au moins deux éléments de commande finals (82, 83) situé sur un côté inférieur du dispositif de maintien (15).

13. Appareil de support de dispositif médical selon la revendication 10, dans lequel la quantité de force d'opération d'un (83) des au moins deux éléments de commande finals (82, 83) de la partie d'entrée situé sur un côté inférieur du dispositif de maintien (15) dans la direction de la force de gravitation est plus petite qu'un moment de chute dû à l'état non équilibré du dispositif de maintien (15).

14. Appareil de support de dispositif médical selon la revendication 10, dans lequel le mécanisme de support comprend une pluralité d'articulations (13) et de bras (12) pour positionner le dispositif de maintien (15) dans un espace tridimensionnel, et comprenant en outre :
un mécanisme de frein (33, 34) disposé sur au moins une des articulations (13a, 13b, 13c) pour commuter l'au moins une articulation entre un état mobile et un état verrouillé,
dans lequel le module de commande (16) est approprié pour commander le mécanisme de frein (33, 34) et dans lequel la pluralité d'éléments de commande finals (82, 83) sont appropriés pour donner des instructions au module de commande (16) pour commuter un état du mécanisme de frein (33, 34).

15. Appareil de support de dispositif médical selon la revendication 14, dans lequel le module de commande (16) fait que le mécanisme de frein (33, 34) commute l'articulation (13) entre l'état mobile et l'état verrouillé lorsque l'opération est libérée par l'un de la pluralité d'éléments de commande finals (82, 83).

16. Appareil de support de dispositif médical selon la revendication 1, dans lequel le mécanisme de support comprend une pluralité d'articulations (13) et de bras (12) pour positionner le dispositif de maintien (15) dans un espace tridimensionnel, et l'appareil de support de dispositif médical comprend en outre :
un mécanisme de frein (33, 34) disposé sur au moins une des articulations pour commuter l'articulation (13) entre un état mobile et un état verrouillé ; et
un mécanisme (35) pour manuellement commuter l'articulation de l'état verrouillé à l'état mobile.
